# EUROPÄISCHE PATENTSCHRIFT

(11) **EP 3 479 918 B1**
(45) Veröffentlichungstag und Bekanntmachung des Hinweises auf die Patenterteilung: **16.11.2022**
(21) Anmeldenummer: 18196875.1
(22) Anmeldetag: 26.09.2018
(51) Int. Cl.: B21C 37/16, A61F 9/007

(54) **VERFAHREN ZUR HERSTELLUNG EINER INNENRÖHRE SOWIE EINE VORRICHTUNG ZUM SCHNEIDEN UND ABSAUGEN VON GEWEBE**
METHOD FOR PRODUCING AN INNER TUBE AND A DEVICE FOR CUTTING AND ASPIRATING TISSUE
PROCÉDÉ DE FABRICATION D'UN TUBE INTERNE AINSI QUE DISPOSITIF DE COUPE ET D'ASPIRATION DE TISSU

(30) Priorität: 26.10.2017 DE 102017219267
(43) Veröffentlichungstag der Anmeldung: 08.05.2019
(73) Patentinhaber: Geuder AG, 69126 Heidelberg (DE)
(72) Erfinder: ENGEL, Stefan, 69126 Heidelberg (DE); DRAHEIM, René, 69207 Sandhausen (DE)
(74) Vertreter: Ullrich & Naumann PartG mbB

(56) Entgegenhaltungen:
- EP-A2- 1 323 483
- DE-A1- 3 032 106
- DE-C1- 3 133 804
- US-A- 5 474 532
- US-A1- 2006 027 009

## Beschreibung

Die Erfindung betrifft ein Verfahren zur Herstellung einer Innenröhre einer Vorrichtung zum Schneiden und Absaugen von Gewebe aus dem menschlichen oder tierischen Auge. Des Weiteren betrifft die Erfindung eine Vorrichtung zum Schneiden und Absaugen von Gewebe aus dem menschlichen oder tierischen Auge mit einer Außenröhre und einer in der Außenröhre angeordneten Innenröhre, wobei die Innenröhre gegenüber der Außenröhre verschiebbar und/oder verdrehbar ist, wobei die Innenröhre und die Außenröhre jeweils mindestens eine Öffnung mit mindestens einer Schneidkante aufweisen und wobei die Schneidkante der Innenröhre mit der Schneidkante der Außenröhre bei einem Verschieben und/oder Verdrehen der Innenröhre schneidend zusammen wirkt.

Grundsätzlich geht es hier um ein chirurgisches Schneidinstrument zur Entnahme von Gewebe aus dem Auge. Mit dem Instrument lässt sich das Gewebe - am bzw. im Auge - schneiden und vom Auge bzw. aus dem Auge heraus absaugen. Im Konkreten kann es sich dabei um ein ganz besonderes Schneidinstrument handeln, mit dem im Rahmen der Vitrektomie der Glaskörper im Auge zerstört bzw. zerkleinert und aus dem Auge entfernt/abgesaugt wird. Auch lassen sich mit diesem Instrument Blut, Blutgerinnsel und bindegewebsähnliche Veränderungen sowie Bereiche der Netzhaut im Rahmen eines Netzhautpeelings entfernen.

Zum Stand der Technik sei lediglich beispielhaft auf die DE 10 2010 050 337 A1 verwiesen. Aus dieser Druckschrift ist eine Vorrichtung bekannt, wobei dort sowohl die äußere Röhre als auch die innere Röhre jeweils zwei seitliche Ausnehmungen mit Doppelschneidfunktion aufweist. Ähnliche Vorrichtungen sind aus der US 5,474,532 und US 5,106,364 bekannt.

Bei diesen Vorrichtungen wird die innere Röhre in Axialrichtung alternierend verschoben, so dass die Schneidkanten der inneren Röhre und der äußeren Röhre schneidend zusammenwirken.

Des Weiteren sind aus der Praxis Vorrichtungen bekannt, bei welchen die innere Röhre gegenüber der äußeren Röhre drehbar ist. Auch bei einer solchen Ausgestaltung wirken die Schneidkanten der inneren Röhre und der äußeren Röhre schneidend zusammen.

Die Innenröhren der voranstehend genannten Vorrichtungen weisen jeweils einen als Ankerrohr dienenden Abschnitt auf, der zur Kopplung mit einem Antriebsmechanismus bzw. zur Befestigung eines Dichtelementes dient. Da der als Innenschneide dienende, distale Endbereich der Innenröhre einen geringeren Durchmesser als das Ankerrohr aufweist, werden zur schrittweisen Reduzierung des Innenrohrdurchmessers sog. Reduzierstücke miteinander verbunden, beispielsweise verschweißt, verlötet oder verklebt. Aufgrund der extrem kleinen Geometrien ist die Herstellung einer zuverlässigen Klebeverbindung jdeoch nur mit sehr großem Aufwand möglich und daher mit hohen Kosten verbunden. Bei einem Verschweißen der Elemente ist problematisch, dass Einschnürungen entstehen. Durch das Verlöten der Elemente wird aufgrund der Zugabe von Flussmittel das Materialgefüge verändert, was ebenfalls von Nachteil ist.

Des Weiteren gehen die voranstehend genannten Herstellungsverfahren mit einem hohen Reinigungsaufwand einher, bspw. Entfetten, Entfernen von Flussmittel und Entfernen von Schmauch.

Ein wesentlicher Nachteil dieser Herstellungsverfahren besteht darin, dass der Durchfluss ("Flow") durch die Innenröhre aufgrund von Überlappungen und Graten an den Verbindungsstellen verringert wird. Auch besteht die Gefahr, dass sich durch das Schweißen eine Wurzel bildet oder dass sich Kleberreste bzw. verhärtetes Flussmittel durch die Endreinigung nicht vollständig entfernen lässt. Alle diese Punkte wirken sich negativ auf das Leistungsmerkmal Flow aus. Schließlich entstehen bei den Rohrübergängen im Inneren zwangsläufig Sacklöcher oder Stufen, die sich negativ auf den Flow in Verbindung mit einem Reflux auswirken.

Weiterhin zeigen die Druckschriften US 2006/027009 A1, DE 31 33 804 C1 und DE 30 32 106 A1 Verfahren zur Herstellung von Kanülen und Röhren.

Der vorliegenden Erfindung liegt die Aufgabe zugrunde, ein Verfahren zur Herstellung einer Innenröhre für eine Vorrichtung zum Schneiden und Absaugen von Gewebe aus dem menschlichen oder tierischen Auge anzugeben, bei dem mit geringem Aufwand eine Innenröhre mit verbessertem Durchfluss herstellbar ist. Des Weiteren soll eine Vorrichtung zum Schneiden und Absaugen von Gewebe aus dem menschlichen oder tierischen Auge mit verbessertem Durchfluss angegeben werden.

Erfindungsgemäß wird die voranstehende Aufgabe betreffend das Herstellungsverfahren durch die Merkmale des Anspruches 1 gelöst. Damit ist ein Verfahren zur Herstellung einer Innenröhre einer Vorrichtung zum Schneiden und Absaugen von Gewebe aus dem menschlichen oder tierischen Auge angegeben, wobei ein Rohrrohling bereitgestellt wird und wobei ein Umformverfahren an dem Rohrrohling durchgeführt wird, so dass mindestens ein Abschnitt mit verringertem Durchmesser erzeugt wird, dadurch gekennzeichnet, dass der Rohrrohling derart dimensioniert ist, dass er in der Vorrichtung als Ankerrohr und/oder Ankerbereich verwendbar ist und dass an dem als Ankerrohr und/oder Ankerbereich dienenden Abschnitt ein Dichtelement angeordnet wird.

In erfindungsgemäßer Weise wird zunächst ein Rohrrohling bereitgestellt. Dieser kann die Form eines Rohres aufweisen und durch ein beliebiges Verfahren hergestellt worden sein. In weiter erfindungsgemäßer Weise ist erkannt worden, dass die zugrunde liegende Aufgabe in verblüffend einfacher Weise gelöst werden kann, indem der Rohrrohling einem Umformverfahren unterzogen wird, so dass mindestens ein Abschnitt mit verringertem Durchmesser an dem Rohrrohling erzeugt wird. Somit ist es nicht mehr notwendig, mehrere Reduzierstücke miteinander zu verbinden, um die unterschiedlichen Durchmesser des Innenrohres zu erhalten. Fügetechniken zur Verbindung von Reduzierstücken und die damit einhergehenden negativen Auswirkungen auf den Durchfluss werden somit vermieden.

Der Begriff "Rohrrohling" ist dabei im weitesten Sinne zu verstehen. Dabei kann es sich um ein Rohr handeln, das zumindest im Wesentlich die Dimensionierung eines Abschnitts des Innenrohres aufweist. Auch wenn das Umformverfahren an dem Rohrrohling durchgeführt worden ist, wird das so entstandene Bauteil im Folgenden als Rohrrohling bezeichnet, gleichwohl dieses nach dem Umformverfahren bereits die endgültige Form des Innenrohres aufweisen kann.

Unter dem "Durchmesser" des Rohrrohlings kann des Innendurchmesser und/oder der Außendurchmesser verstanden werden.

In besonders vorteilhafter Weise wird an unterschiedlichen Abschnitten des Rohrrohlings das Umformverfahren durchgeführt, so dass an dem Rohrrohling aneinander angrenzende Abschnitte mit jeweils geringer werdendem Durchmesser erzeugt werden. Somit ist es möglich, den Rohrrohling schrittweise im Durchmesser zu verkleinern, ohne ein Reduzierstück einbringen zu müssen, wodurch der Durchfluss erheblich verbessert wird.

Bei dem Umformverfahren kann es sich um ein Zugdruckumformverfahren oder um ein Ziehverfahren handeln. In besonders vorteilhafter Weise kann es sich um ein Durchziehverfahren handeln. Im Grunde kann jedes Umformverfahren gewählt werden, mit dem der Durchmesser des Innenrohlings in der gewünschten Weise verändert bzw. verringert werden kann.

Zur weiteren Vereinfachung des Verfahrens ist der bereitgestellte Rohrrohling bereits derart dimensioniert, dass er in der Vorrichtung als Ankerrohr verwendbar ist. Hierzu kann der Rohrrohling einen entsprechend großen Durchmesser sowie die notwendige Wanddicke bzw. Materialstärke aufweisen, dass er als Ankerrohr bzw. Ankerbereich dienen kann. Das Umformverfahren wird sodann nur in bzw. an den Abschnitten des Rohrrohlings durchgeführt, die nicht als Ankerrohr dienen sollen. Des Weiteren wird an dem als Ankerrohr dienenden Abschnitt ein Dichtelement angeordnet.

In weiter vorteilhafter Weise kann an dem Abschnitt mit dem geringsten Durchmesser mindestens eine Schneidkante ausgebildet werden. Beispielsweise kann der Rohrrohling an dem distalen Ende offen sein, wobei der Rand der distalen Öffnung als Schneidkante ausgebildet wird. Diese dient dazu, mit einer Schneidkante eines Außenrohres bei einer Bewegung des Innenrohres gegenüber dem Außenrohr schneidend zusammen zu wirken. Alternativ oder zusätzlich ist es denkbar, dass an dem Abschnitt mit dem geringsten Durchmesser mindestens eine seitliche Öffnung mit mindestens einer Schneidkante erzeugt wird.

In besonders vorteilhafter Weise kann der Abschnitt mit dem geringsten Durchmesser einen Durchmesser von 20 Gauge bis 29 Gauge aufweisen. Beispielsweise 20 Gauge oder 23 Gauge oder 25 Gauge oder 27 Gauge oder 29 Gauge. Dadurch ist das distale Ende des Innenrohres in idealer Weise zur Anwendung in der Ophthalmologie geeignet.

Die zugrunde liegende Aufgabe wird des Weiteren durch die Vorrichtung mit den Merkmalen des nebengeordneten Anspruch 8 gelöst. Danach ist eine Vorrichtung zum Schneiden und Absaugen von Gewebe aus dem menschlichen oder tierischen Auge mit einer Außenröhre und einer in der Außenröhre angeordneten Innenröhre, wobei die Innenröhre gegenüber der Außenröhre verschiebbar und/oder verdrehbar ist, wobei die Innenröhre und die Außenröhre jeweils mindestens eine Öffnung mit mindestens einer Schneidkante aufweisen und wobei die Schneidkante der Innenröhre mit der Schneidkante der Außenröhre bei einem Verschieben und/oder Verdrehen der Innenröhre schneidend zusammen wirkt, dadurch gekennzeichnet, dass die Innenröhre mit einem Verfahren nach einem der Ansprüche 1 bis 9 hergestellt ist.

In erfindungsgemäße Weise ist erkannt worden, dass sich die Verbindung von Reduzierstücken mittels Löten, Schweißen oder Kleben negativ auf den Durchfluss und somit die Leistungsfähigkeit der Vorrichtung auswirkt. Des Weiteren ist erkannt worden, dass diese Probleme vermieden werden können, indem eine Innenröhre verwendet wird, die mit einem Verfahren nach einem der Ansprüche 1 bis 7 hergestellt ist.

An dieser Stelle wird darauf hingewiesen, dass das erfindungsgemäße Verfahren auch vorrichtungsgemäße Ausprägungen aufweisen kann. Die voranstehend in Bezug auf das Verfahren genannten Merkmale und Vorteile können somit ausdrücklich Teil der beanspruchten Vorrichtung sein und stellen daher auch einen Bestandteil der Offenbarung in Bezug auf die erfindungsgemäße Vorrichtung dar.

Es gibt nun verschiedene Möglichkeiten, die Lehre der vorliegenden Erfindung in vorteilhafter Weise auszugestalten und weiterzubilden. Dazu ist einerseits auf die dem Anspruch 1 nachgeordneten Ansprüche und andererseits auf die nachfolgende Erläuterung bevorzugter Ausführungsbeispiele der Erfindung anhand der Zeichnung zu verweisen. In Verbindung mit der Erläuterung der bevorzugten Ausführungsbeispiele der Erfindung anhand der Zeichnung werden auch im Allgemeinen bevorzugte Ausgestaltungen und Weiterbildungen der Lehre erläutert. In der Zeichnung zeigen
- Fig. 1: in einer schematischen Darstellung eine aus dem Stand der Technik bekannte Innenröhre für eine Vorrichtung zum Schneiden und Absaugen von Gewebe aus dem Auge,
- Fig. 2: in einer schematischen, vergrößerten Darstellung einen Ausschnitt aus der Darstellung gemäß Fig. 1, und
- Fig. 3: in einer schematischen Darstellung, ein Ausführungsbeispiel einer nac dem erfindungsgemäßen Verfahren hergestellten Innenröhre.

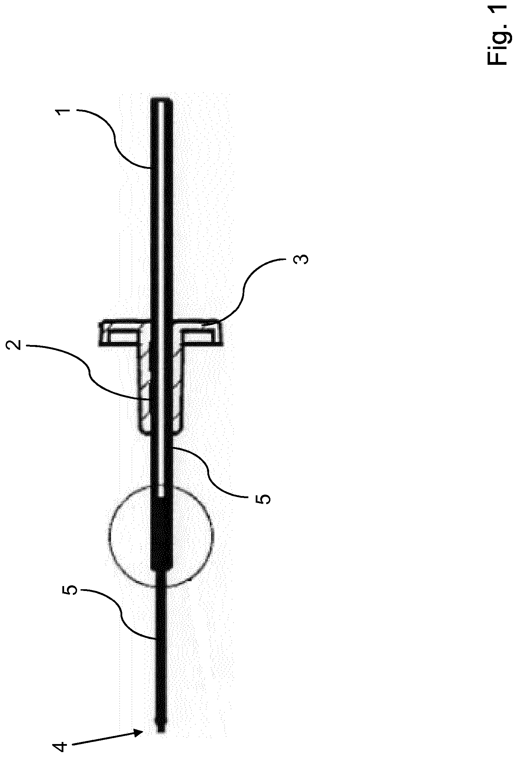

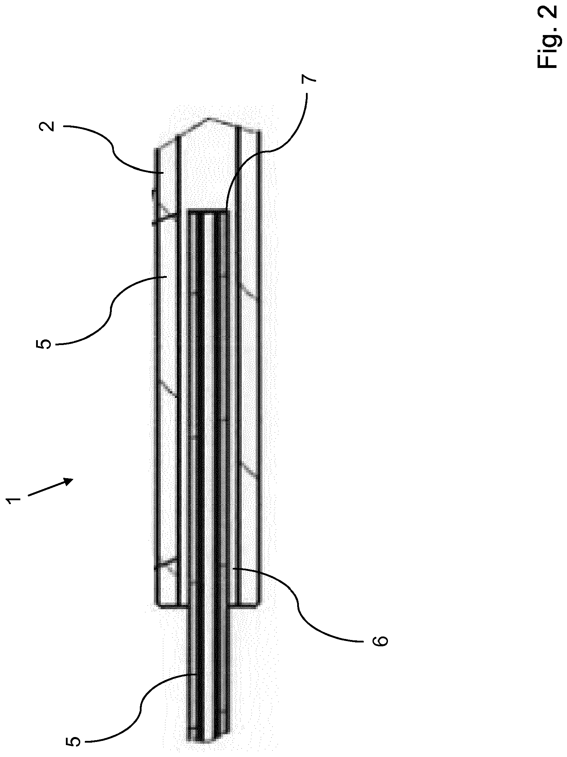

In den Fig. 1 und 2 ist eine aus dem Stand der Technik bekannte Innenröhre 1 für eine Vorrichtung zum Schneiden und Absaugen von Gewebe dargestellt. Die Innenröhre 1 umfasst einen Ankerbereich 2, an dem ein Hartteil 3 des Ankers angeordnet ist. Zum distalen Ende 4 der Innenröhre 1 hin nimmt der Durchmesser der Innenröhre 1 ab. Hierzu ist die Innenröhre 1 aus mehreren Reduzierstücken 5 ausgebildet, die miteinander verlötet, verschweißt oder verklebt sind.

In Fig. 2 in deutlich zu erkennen, dass sich an den Übergängen der Reduzierstücke 5 Sacklöcher 6 oder Stufen 7 bilden, die sich negativ auf den Durchfluss in Verbindung mit einem Reflux auswirken.

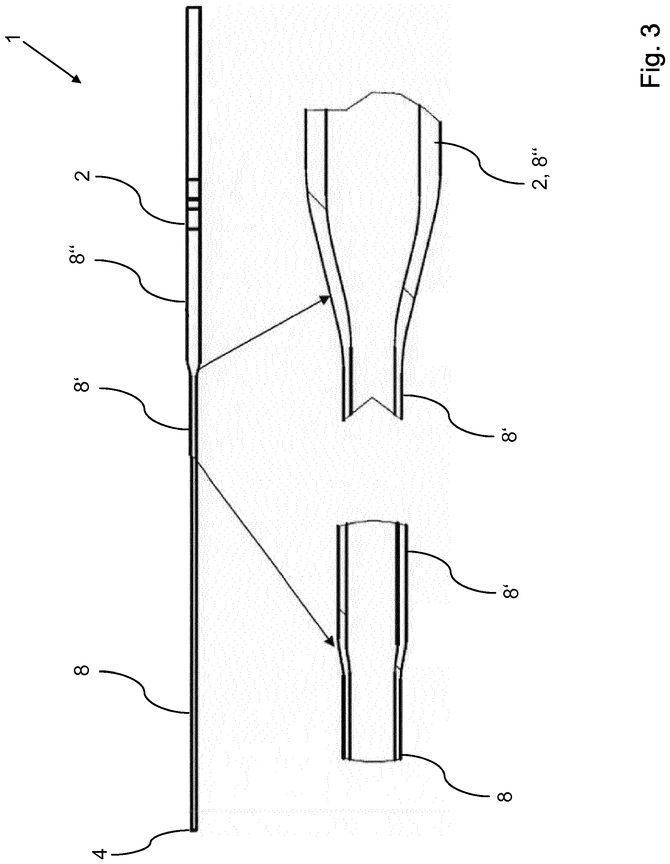

In Fig. 3 ist eine mit dem erfindungsgemäßen Verfahren hergestellte Innenröhre 1 gezeigt. Die Innenröhre 1 wird aus einem Rohrrohling hergestellt, indem dieser einem Umformverfahren unterzogen wird, so dass sich der Durchmesser der Innenröhre 1 bzw. des Rohrrohlings verringert. Auf diese Weise sind in dem hier dargestellten Ausführungsbeispiel drei Abschnitte 8, 8', 8" mit unterschiedlichem Durchmesser an der Innenröhre 1 realisiert. Der Durchmesser nimmt dabei von dem proximalen Abschnitt 8" bis hin zu dem distalen Abschnitt 8 ab.

Den in Fig. 3 dargestellten vergrößerten Ausschnitten der Innenröhre 1 ist zu entnehmen, dass der Übergang zwischen den Abschnitten 8, 8', 8" keine Grate, Sacklöcher, Stufen o.ä. aufweist, was sich negativ auf den Durchfluss auswirken würde. An dem distalen Ende 4 der Innenröhre ist eine in Fig. 3 nicht dargestellte Schneidkante ausgebildet, die mit der Schneidkante einer Außenröhre schneidend zusammenwirken kann.

Die in Fig. 3 gezeigte Innenröhre 1 wird hergestellt, indem ein Rohrrohling bereitgestellt und einem Umformverfahren unterzogen wird, so dass die Abschnitte 8, 8' mit verringertem Durchmesser erzeugt werden. In besonders vorteilhafter Weise kann der Rohrrohling derart dimensioniert sein, dass er bereits als Ankerbereich 2 dienen kann. Somit wird der Ankerbereich 2 realisiert, indem der Durchmesser des Rohrrohlings in diesem Abschnitt 8" nicht verringert wird. Bei dem Umformverfahren kann es sich beispielsweise um ein Ziehverfahren handeln. An dem distalen Abschnitt 8 des Rohrrohlings bzw. der Innenröhre 1 kann mindestens eine Schneidkante erzeugt werden, so dass diese mit einer Schneidkante der Außenröhre schneidend zusammenwirkt, wenn die Innenröhre 1 gegenüber der Außenröhre bewegt wird.

Hinsichtlich weiterer vorteilhafter Ausgestaltungen des erfindungsgemäßen Verfahrens und der erfindungsgemäßen Vorrichtung wird zur Vermeidung von Wiederholungen auf den allgemeinen Teil der Beschreibung sowie auf die beigefügten Ansprüche verwiesen.

Schließlich sei ausdrücklich darauf hingewiesen, dass die voranstehend beschriebenen Ausführungsbeispiele des erfindungsgemäßen Verfahrens und der erfindungsgemäßen Vorrichtung lediglich zur Erörterung der beanspruchten Lehre dienen, diese jedoch nicht auf die Ausführungsbeispiele einschränken.

### Bezugszeichenliste

- 1: Innenröhre
- 2: Ankerbereich
- 3: Hartteil
- 4: distales Ende (Innenröhre)
- 5: Reduzierstück
- 6: Sackloch
- 7: Stufe
- 8, 8', 8": Abschnitt (Innenröhre)

## Patentansprüche

1. Verfahren zur Herstellung einer Innenröhre (1) einer Vorrichtung zum Schneiden und Absaugen von Gewebe aus dem menschlichen oder tierischen Auge, wobei ein Rohrrohling bereitgestellt wird und wobei ein Umformverfahren an dem Rohrrohling durchgeführt wird, so dass mindestens ein Abschnitt (8, 8') mit verringertem Durchmesser erzeugt wird,
**dadurch gekennzeichnet, dass** der Rohrrohling derart dimensioniert ist, dass er in der Vorrichtung als Ankerrohr und/oder Ankerbereich (2) verwendbar ist und dass an dem als Ankerrohr und/oder Ankerbereich (2) dienenden Abschnitt (8") ein Dichtelement angeordnet wird,

2. Verfahren nach Anspruch 1, **dadurch gekennzeichnet, dass** an unterschiedlichen Abschnitte (8, 8', 8") des Rohrrohlings das Umformverfahren durchgeführt wird, so dass an dem Rohrrohling sich aneinander anschließende Abschnitte (8, 8', 8") mit jeweils geringer werdendem Durchmesser erzeugt werden.

3. Verfahren nach Anspruch 1 oder 2, **dadurch gekennzeichnet, dass** es sich bei dem Umformverfahren um ein Zugdruckumformverfahren handelt.

4. Verfahren nach einem der Ansprüche 1 bis 3, **dadurch gekennzeichnet, dass** es sich bei dem Umformverfahren um ein Ziehverfahren, insbesondere ein Durchziehverfahren, handelt.

5. Verfahren nach einem der Ansprüche 1 bis 4, **dadurch gekennzeichnet, dass** an dem Abschnitt (8) mit dem geringsten Durchmesser mindestens eine Schneidkante ausgebildet wird.

6. Verfahren nach einem der Ansprüche 1 bis 5, **dadurch gekennzeichnet, dass** an dem Abschnitt (8) mit dem geringsten Durchmesser mindestens eine seitliche Öffnung mit mindestens einer Schneidkante ausgebildet wird.

7. Verfahren nach einem der Ansprüche 1 bis 6, **dadurch gekennzeichnet, dass** der Abschnitt (8) mit dem geringsten Durchmesser einen Durchmesser von 20 Gauge bis 29 Gauge aufweist, beispielsweise 20 Gauge oder 23 Gauge oder 25 Gauge oder 27 Gauge oder 29 Gauge.

8. Vorrichtung zum Schneiden und Absaugen von Gewebe aus dem menschlichen oder tierischen Auge mit einer Außenröhre und einer in der Außenröhre angeordneten Innenröhre (1), wobei die Innenröhre (1) gegenüber der Außenröhre verschiebbar und/oder verdrehbar ist, wobei die Innenröhre (1) und die Außenröhre jeweils mindestens eine Öffnung mit mindestens einer Schneidkante aufweisen und wobei die Schneidkante der Innenröhre (1) mit der Schneidkante der Außenröhre bei einem Verschieben und/oder Verdrehen der Innenröhre (1) schneidend zusammen wirkt,
**dadurch gekennzeichnet, dass** die Innenröhre (1) mit einem Verfahren nach einem der Ansprüche 1 bis 7 hergestellt ist.

## Claims

1. Method for producing an inner tube (1) of an apparatus for cutting and drawing off tissue from the human or animal eye, wherein a tube blank is provided and wherein a shaping method is carried out on the tube blank so that at least one portion (8, 8') having a reduced diameter is produced,
**characterised in that** the tube blank has such dimensions that it can be used in the apparatus as an anchor tube and/or anchor region (2) and **in that** a sealing element is arranged on the portion (8") which acts as an anchor tube and/or anchor region (2).

2. Method according to claim 1, **characterised in that** the shaping method is carried out at various portions (8, 8', 8") of the tube blank so that portions (8, 8', 8") which adjoin each other and which have a diameter which becomes increasingly small are formed on the tube blank.

3. Method according to claim 1 or 2, **characterised in that** the shaping method is a tension and compression shaping method.

4. Method according to any one of claims 1 to 3, **characterised in that** the shaping method is a drawing method, in particular a draw-through method.

5. Method according to any one of claims 1 to 4, **characterised in that** at least one cutting edge is formed on the portion (8) with the smallest diameter.

6. Method according to any one of claims 1 to 5, **characterised in that** at least one lateral opening having at least one cutting edge is formed on the portion (8) with the smallest diameter.

7. Method according to any one of claims 1 to 6, **characterised in that** the portion (8) with the smallest diameter has a diameter of from 20 Gauge to 29 Gauge, for example, 20 Gauge or 23 gauge or 25 Gauge or 27 Gauge or 29 Gauge.

8. Apparatus for cutting and drawing off tissue from the human or animal eye, having an outer tube and an inner tube (1) which is arranged in the outer tube, wherein the inner tube (1) can be displaced and/or rotated with respect to the outer tube, wherein the inner tube (1) and the outer tube each have at least one opening having at least one cutting edge and wherein the cutting edge of the inner tube (1) cooperates in a cutting manner with the cutting edge of the outer tube when the inner tube (1) is displaced and/or rotated,
**characterised in that** the inner tube (1) is produced with a method according to any one of claims 1 to 7.

## Revendications

1. Procédé de fabrication d'un tube intérieur (1) d'un dispositif de coupe et d'aspiration de tissu à partir de l'œil humain ou animal, une ébauche de tube étant fournie et un procédé de formage étant réalisé sur l'ébauche de tube de telle sorte qu'au moins un tronçon (8, 8') de diamètre réduit est produit,
**caractérisé en ce que** l'ébauche de tube est dimensionnée de telle sorte qu'elle peut être utilisée en tant que tube d'ancrage et/ou zone d'ancrage (2), et **en ce qu'**un élément d'étanchéité est disposé sur le tronçon (8") servant de tube d'ancrage et/ou de zone d'ancrage (2).

2. Procédé selon la revendication 1, **caractérisé en ce que** le procédé de formage est effectué sur différents tronçons (8, 8', 8") de l'ébauche de tube de telle sorte que des tronçons (8, 8', 8") raccordés les uns aux autres ayant respectivement un diamètre qui diminue sont produits sur l'ébauche de tube.

3. Procédé selon la revendication 1 ou 2, **caractérisé en ce que**, concernant le procédé de formage, il s'agit d'un procédé de formage par traction et compression.

4. Procédé selon l'une des revendications 1 à 3, **caractérisé en ce que**, concernant le procédé de formage, il s'agit d'un procédé de formage par étirage, en particulier d'un procédé par étirage continu.

5. Procédé selon l'une des revendications 1 à 4, **caractérisé en ce qu'**au moins une arête de coupe est constituée sur le tronçon (8) ayant le plus petit diamètre.

6. Procédé selon l'une des revendications 1 à 5, **caractérisé en ce qu'**au moins une ouverture latérale ayant au moins une arête de coupe est constituée sur le tronçon (8) ayant le plus petit diamètre.

7. Procédé selon l'une des revendications 1 à 6, **caractérisé en ce que** le tronçon (8) ayant le plus petit diamètre présente un diamètre de 20 gauge à 29 gauge, par exemple de 20 gauge ou 23 gauge ou 25 gauge ou 27 gauge ou 29 gauge.

8. Dispositif de coupe et d'aspiration de tissu à partir de l'œil humain ou animal avec un tube extérieur et un tube intérieur (1) disposé dans le tube extérieur, le tube intérieur (1) pouvant coulisser et/ou tourner par rapport au tube extérieur, le tube intérieur (1) et le tube extérieur comportant respectivement au moins une ouverture ayant au moins une arête de coupe, et l'arête de coupe du tube intérieur (1) coopérant dans une action de coupe avec l'arête de coupe du tube extérieur lors d'un coulissement et/ou d'une rotation du tube intérieur (1),
**caractérisé en ce que** le tube intérieur (1) est fabriqué avec un procédé selon l'une des revendications 1 à 7.
